# NOUVEAU FASCICULE DE BREVET EUROPEEN

(11) **EP 1 935 455 B2**
(45) Date de publication et mention de la décision concernant l'opposition: **28.03.2018**
(45) Mention de la délivrance du brevet: 31.03.2010
(21) Numéro de dépôt: 07123806.7
(22) Date de dépôt: 20.12.2007
(51) Int. Cl.: A61Q 5/06, A61K 8/41, A61K 8/44, A61K 8/73

(54) **Composition de teinture directe comprenant un tensio-actif cationique, un biohétéropolysaccharide, un tensio-actif amphotère et un colorant direct**
Zusammensetzung zur Direktfärbung enthaltend ein kationisches Tensid, ein Bioheteropolysaccharid, ein amphoteres Tensid und einen direktziehenden Farbstoff
Direct dye composition comprising a cationic surfactant, a bioheteropolysaccharide, an amphoteric surfactant and a direct dye

(30) Priorité: 21.12.2006 FR 0655808
(43) Date de publication de la demande: 25.06.2008
(73) Titulaire: L'Oréal, 75008 Paris (FR)
(72) Inventeur: Huet, Nathalie, 75012, PARIS (FR); Audousset, Marie-Pascale, 92600, ASNIERES (FR)
(74) Mandataire: Duvert, Sandra

(56) Documents cités:
- EP-A- 1 559 403
- EP-A1- 1 224 927
- EP-A1- 1 905 421
- EP-A2- 0 834 303
- CA-A- 1 222 208
- DE-U1- 20 011 145
- FR-A1- 2 548 895
- JP-A- 2006 267 046
- US-A- 4 904 275
- US-A- 5 196 029

## Description

La présente invention concerne une composition de teinture directe des fibres kératiniques, en particulier des fibres kératiniques humaines.

Il est connu de teindre les fibres kératiniques et en particulier les cheveux humains, avec des compositions de teinture contenant des colorants directs, en particulier des colorants benzéniques nitrés, des colorants azoïques acides, des colorants azoïques cationiques, des colorants anthraquinoniques, des colorants naturels.

Ces colorations peuvent être réalisées par application directe sur les fibres kératiniques de la composition contenant le ou les colorants directs ou par application d'un mélange réalisé extemporanément d'une composition contenant le ou les colorants directs avec une composition contenant un agent décolorant oxydant qui est de préférence l'eau oxygénée. On parle alors, dans ce dernier cas, de coloration directe éclaircissante.

Il est déjà connu de réaliser des compositions de colorations directes des fibres kératiniques en associant aux colorants directs des tensio-actifs et/ou des épaississants pour améliorer les propriétés physiques et/ou les colorations obtenues.

Il est notamment connu du document FR 2 548 895 des compositions comprenant un colorant direct et une gomme de xanthane pour obtenir un meilleur épaississement des compositions de colorations directes, ces compositions pouvant contenir ou non des tensio-actifs.

Ces compositions ne donnent cependant pas des propriétés cosmétiques et/ou tinctoriales totalement satisfaisantes, notamment en ce qui concerne l'intensité des colorations et leurs sélectivités représentatives d'écarts de couleur entre différentes parties d'un cheveu ou des cheveux suivant les degré de sensibilisation.

Ainsi, le but de la présente invention est de développer de nouvelles compositions de teinture directe des fibres kératiniques telles que les cheveux qui permettent de résoudre ce problème technique. En particulier, l'invention cherche à obtenir des compositions qui permettent d'obtenir des nuances intenses et peu sélectives, le cheveu ayant, par ailleurs de bonnes propriétés cosmétiques.

Ainsi, ce but est atteint avec la présente invention qui a pour objet une composition de coloration des fibres kératiniques comprenant au moins un colorant direct, au moins un biohétéropolysaccharide, au moins un tensio-actif cationique et au moins un tensio-actif amphotère choisi parmi les alkyl (C₈-C₂₀) bétaïnes, les alkyl (C₈-C₂₀) amidoalkyl (C₁-C₆) bétaïnes ou les alkyl (C₈-C₂₀), amidoalkyl (C₁-C₆) sulfobétaïnes.

Dans la composition de l'invention, les colorants directs peuvent être choisis parmi tous les colorants directs connus de la technique pour la coloration des fibres kératiniques, notamment les cheveux.

Les colorants directs utilisables selon l'invention sont choisis de préférence parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention, on peut citer de manière non limitative les composés suivants:
• 1,4-diamino-2-nitrobenzène
• 1-amino-2 nitro-4-β-hydroxyéthylaminobenzène
• 1-amino-2 nitro-4-bis(β-hydroxyéthyl)-aminobenzène
• 1,4-Bis(β-hydroxyéthylamino)-2-nitrobenzène
• 1-β-hydroxyéthylamino-2-nitro-4-bis-(β-hydroxyéthylamino)-benzène
• 1-β-hydroxyéthylamino-2-nitro-4-aminobenzène
• 1-β-hydroxyéthylamino-2-nitro-4-(éthyl)(β-hydroxyéthyl)-aminobenzène
• 1-amino-3-méthyl-4-β-hydroxyéthylamino-6-nitrobenzène
• 1-amino-2-nitro-4-β-hydroxyéthylamino-5-chlorobenzène
• 1,2-Diamino-4-nitrobenzène
• 1-amino-2-β-hydroxyéthylamino-5-nitrobenzène
• 1,2-Bis-(β-hydroxyéthylamino)-4-nitrobenzène
• 1-amino-2-tris-(hydroxyméthyl)-méthylamino-5-nitrobenzène
• 1-Hydroxy-2-amino-5-nitrobenzène
• 1-Hydroxy-2-amino-4-nitrobenzène
• 1-Hydroxy-3-nitro-4-aminobenzène
• 1-Hydroxy-2-amino-4,6-dinitrobenzène
• 1-β-hydroxyéthyloxy-2-β-hydroxyéthylamino-5-nitrobenzène
• 1-Méthoxy-2-β-hydroxyéthylamino-5-nitrobenzène
• 1-β-hydroxyéthyloxy-3-méthylamino-4-nitrobenzène
• 1-β,γ-dihydroxypropyloxy-3-méthylamino-4-nitrobenzène
• 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoïques cationiques décrits dans les demandes de brevets WO 95/15144, WO-95/01772 et EP-714954 dont le contenu fait partie intégrante de l'invention.
Parmi ces composés on peut tout particulièrement citer les colorants suivants:
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3° édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Les colorants directs peuvent être fluorescents. A titre de colorants fluorescents, on peut citer les composés de structures I ou II suivantes : 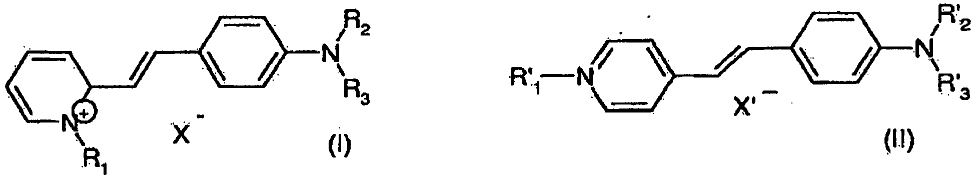 avec R₁, R₂, R₃ ; R'₁, R'₂, R'₃ désignant un radical alkyle en C₁-C₁₀ et de préférence en C₁-C₄ X⁻ désignant un contre-ion d'un acide minéral et/ou organique.

Le ou les colorants directs représentent de préférence de 0,001 à 20% en poids du poids total de la composition et préférentiellement de 0,005 à 10% en poids.

Au sens de la présente invention, on entend par bio hétéropolysaccharides, des substances synthétisées par fermentation de sucres par des micro-organismes. Les biohétéropolysaccharides ont en particulier souvent des unités choisies parmi les unités mannoses, glucoses et acide glucuroniques ou galacturoniques dans leur chaîne, ces unités étant éventuellement acylées.

On peut notamment citer les gommes de xanthane produites par la bactérie *xanthomonas campestri* et les mutants et variants de celle-ci.

Ces gommes de xanthane ont généralement un poids moléculaire compris entre 1 000 000 et 50 000 000.

On peut aussi citer les gommes de scléroglucane produites par *Sclerotium rolfsii,* les gommes de gellane produites par *Pseudomonas elodea* ou *Sphingomonias,* les gommes de pullulane produites par *Aureobacidium pullulens,* les gommes de Curdlar produites par les alcaligènes de type *Faecalis myxogènes*, les gommes de xanthanes produits par de nombreux organismes dont *Leuconostoc mesenteroides* et *Leuconostoc dextrantum*, les gommes de grifolane produites par *Grifola frondara,* les gommes de lentinane produites par Lentinus e dodes, les gommes de Schizophyllane produites par *Schizophyllum commine,* les gommes de spirulinane produites par Spirulina sybsyla et les gommes de krestine produites par *Coriates versicolor.*

De préférence, on utilise les gommes de xanthane et de scléroglucane. Encore plus préférentiellement, on utilise les gommes de scléroglucane.

Le biohétéropolysaccharide est en général compris dans la composition de coloration dans des quantités variant de 0.01 à 10 % en poids du poids total de la composition, de préférence entre 0.1 et 4 %.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

Selon l'invention, les tensioactifs cationiques sont non polymériques, c'est à dire ne sont pas obtenus par polymérisation de monomères autres que les oxyde d'alkylènes ou par greffage de groupes cationiques sur des pigments naturels existants.

A titre d'amines grasses, on peut notamment citer les alkyl amido amines telles que par exemple les alkyl (C₈-C₃₀) amido dialkyl (C₁-C₆)amines et en particulier la stéaramido propyl diméthylamine (MACKINE 301 proposé par MAC INTYRE).

A titre de sels d'ammonium quaternaire, on peut notamment citer, par exemple :
- ceux qui présentent la formule générale (V) suivante : 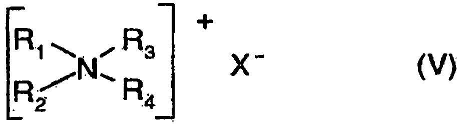 dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle en C1-C30, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline comme, par exemple, ceux de formule (VI) suivante : 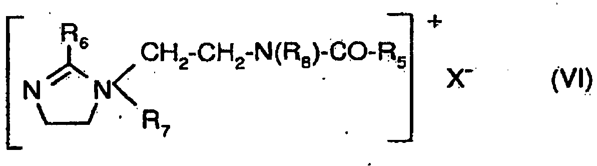 dans laquelle R₅ représente un radical alcényle ou alkyle comportant de 8 à 30 atomes de carbone par exemple dérivés des acides gras du suif ou du coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27 (CTFA 2002), le Quaternium-87 (CTFA 2002) ou le Quaternium-83 (CTFA 2002) commercialisés sous les dénominations "VARISOFT®" W575PG par la société GOLDSCHMIDT,
- les sels de diammonium quaternaire de formule (VII) : 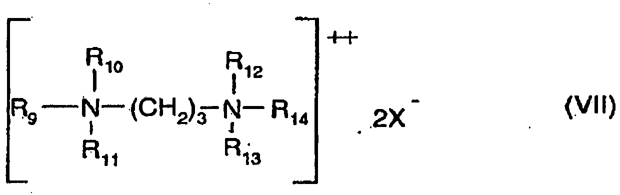 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X⁻ est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
- les sels d'ammonium quaternaire comprenant au moins une fonction ester, tels que ceux de formule (VIII) suivante : 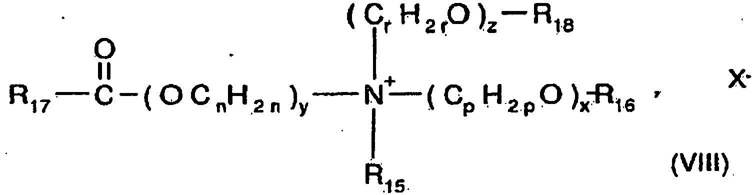 dans laquelle :
   R₁₅ est choisi parmi les radicaux alkyles en C₁-C₆ et les radicaux hydroxyalkyles ou dihydroxyalkyles en C₁-C₆ ;
   R₁₆ est choisi parmi :
      - le radical 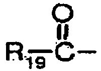
      - les radicaux R₂₀ hydrocarbonés en C₁-C₂₂, linéaires ou ramifiés, saturés ou insaturés,
      - l'atome d'hydrogène,
   R₁₇ est choisi parmi :
      - le radical 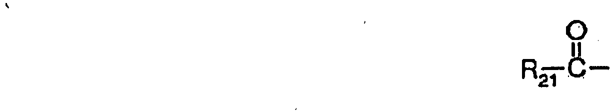
      - les radicaux R₂₂ hydrocarbonés en C₁-C₆, linéaires ou ramifiés, saturés ou insaturés,
      - l'atome d'hydrogène,
   R₁₇, R₁₉ et R₂₁, identiques ou différents, sont choisis parmi les radicaux hydrocarbonés en C₇-C₂₁, linéaires ou ramifiés, saturés ou insaturés ;
      r, n et p, identiques ou différents, sont des entiers valant de 2 à 6 ;
      y est un entier valant de 1 à 10 ;
      x et z, identiques ou différents, sont des entiers valant de 0 à 10;
      X⁻ est un anion simple ou complexe, organique ou inorganique ;
sous réserve que la somme x + y + z vaut de 1 à 15, que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés, et plus particulièrement linéaires.

De préférence, R₁₅ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone, ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, n et p, identiques ou différents, valent 2 ou 3, et encore plus particulièrement sont égaux à 2.

L'anion X⁻ est de préférence un halogénure (chlorure, bromure ou iodure) ou un alkyl(C₁-C₄)Sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₁₅ désigne un radical méthyle ou éthyle,
- x et y sont égaux à 1 ;
- z est égal à 0 ou 1 ;
- r, n et pt sont égaux à 2 ;
- R₁₆ est choisi parmi :
- le radical 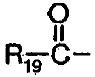
- les radicaux méthyle, éthyle ou hydrocarbonés en C₁₄-C₂₂,
- l'atome d'hydrogène ;
- R₁₈ est choisi parmi :
- le radical 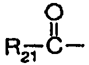
   - l'atome d'hydrogène ;
   - R₁₇, R₁₉ et R₂₁, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (VIII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyl-diisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-GOLDSCHMIDT.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange comprenant 15 à 30% en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium comprenant au moins une fonction ester décrits dans les brevets US-A-4874554 et US-A-4137180.

Parmi les sels d'ammonium quaternaire mentionnés ci-dessus, on préfère utiliser ceux répondant à la formule (V) ou de formule (VIII). On peut notamment citer d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthyl-stéarylammonium ou encore, d'autre part, le chlorure de palmitylamidopropyltriméthylammonium ou le chlorure de stéaramidopropyldiméthyl-(myristyl acétate)-ammonium correspondant au QUATERNIUM-70 (CTFA 2002) commercialisé sous la dénomination CERAPHYL® 70 par la société ISP.

Les tensioactifs cationiques particulièrement préférés dans la composition de l'invention sont choisis parmi les sels d'ammonium quaternaire, et en particulier parmi le chlorure de béhényltriméthylammonium, le chlorure de cétyltriméthylammonium, le quaternium-83, le quaternium-87, le chlorure de béhénylamidopropyl 2,3-dihydroxypropyl diméthyl ammonium, le chlorure de palmitylamidopropyltriméthylammonium et la stéaramidopropyldiméthylamine.

La composition selon l'invention comprend de préférence le ou les tensioactifs cationiques en une quantité allant de 0,01 à 10 % en poids, de préférence de 0,1 à 4 % en poids par rapport au poids total de la composition.

De préférence, le rapport pondéral tensioactifs cationiques/le ou les biopolysaccharides est supérieur à 1 et idéalement compris entre 1 et 10.

Les tensio-actifs amphotères contenus dans la composition de l'invention sont choisis parmi les alkyl (C₈-C₂₀) bétaïnes, les alkyl (C₈-C₂₀) amidoalkyl (C₁-C₆) bétaïnes ou les alkyl (C₈-C₂₀) amidoalkyl (C₁-C₆) sulfobétaïnes.

La composition selon l'invention comprend de préférence le ou les tensioactifs amphotères en une quantité allant de 0,01 à 20 % en poids, de préférence de 5 à 15 % en poids par rapport au poids total de la composition.

La composition de l'invention peut comprendre d'autres tensio-actifs, par exemples des tensio-actifs non ioniques. A titre d'exemple, on peut citer les tensio-actifs non ioniques décrits dans "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. De préférence, on utilise les alkylpolyglycosides ou les alcools gras oxyalkylénés.

La composition de la présente invention peut de plus contenir des bases d'oxydations et des coupleurs classiquement utilisés pour la coloration par oxydation.

A titre d'exemple, on peut citer les paraphénylènediamines, les bis-phénylalkylènediamines, les para-aminophénols, les ortho-aminophénols, les bases hétérocycliques et leurs sels d'addition.

Les coupleurs sont par exemple les coupleurs métaphénylènediamines, les coupleurs méta-aminophénols, les coupleurs métadiphénols, les coupleurs naphtaléniques, les coupleurs hétérocycliques et leurs sels d'addition.

Lorsqu'ils sont présents, les bases et les coupleurs sont chacun généralement présents en quantité comprise entre 0,001 et 10 % en poids environ du poids total de la composition tinctoriale, de préférence entre 0,005 et 6 %.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Pour la teinture des fibres kératiniques humaines, le milieu de teinture est un milieu cosmétique approprié.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, ou zwittérioniques ou leurs mélanges, des polymères anioniques, cationiques, non-ioniques, amphotères, zwittérioniques ou leurs mélanges, des agents épaississants autres que les biohétérosaccharides utiles dans la présente invention, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 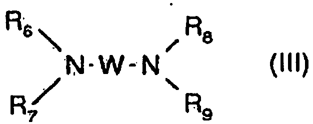 dans laquelle W est un reste propylène éventuellement substitué par un groupement hydroxyle ou un radical alkyle en C₁-C₄ R₆, R₇, R₈ et R₉, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a aussi pour objet un procédé de teinture directe qui comprend l'application d'une composition telle que définie précédemment sur les fibres kératiniques. Après un temps de pause, les fibres kératiniques sont rincées laissant apparaître des fibres colorées. Le temps de pause est généralement compris entre 3 à 50 minutes environ, de préférence 5 à 30 minutes environ.

Lorsque la composition tinctoriale comprend une base d'oxydation et/ou un coupleur, la composition tinctoriale peut alors contenir un agent oxydant. Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydoréductases à 2 électrons telles que les uricases et les oxygénases à 4 électrons comme les laccases. Le peroxyde d'hydrogène est particulièrement préféré.

L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

### EXEMPLES

Les compositions suivantes ont été préparées, les quantités indiquées sauf indication contraire dans des quantités pondérales.

### Exemple 1

| | Exemple 1 |
|---|---|
| 4-AMINO-3-NITROPHENOL | 0,145 |
| 3-NITRO-p-HYDROXYETHYLAMINOPHENOL | 1,35 |
| HC RED NO. 7 | 0,452 |
| HC RED NO. 3 | 0,18 |
| BASIC RED 51 | 0,04 |
| BASIC ORANGE 31 | 0,02 |
| CHLORHEXIDINE DIHYDROCHLORIDE | 0,05 |
| METHYLPARABEN | 0,3 |
| SCLEROTIUMGUM | 1 |
| AMODIMETHICONE (and) TRIDECETH-6 (and) CETRIMONIUM CHLORIDE | 1,8 |
| PROPYLENE GLYCOL | 2 |
| CETEARYL ALCOHOL | 7 |
| BEHENTRIMONIUM CHLORIDE | 4 |
| COCAMIDOPROPYL BETAINEE | 10 |
| ETHANOLAMINE | QS pH 6.7 |
| CITRIC ACID | |
| AQUA | QSP |

Cette composition est ensuite appliquée sur des mèches de cheveux gris naturels à 90% de blancs à température ambiante. Après 30 minutes, les mèches de cheveux sont rincées à l'eau courante puis séchées.

Les mèches ont été teintes dans une nuance Auburn intense et peu sélective. Les cheveux sont doux.

### Exemple 2

| | Exemple 2 |
|---|---|
| 3-METHYLAMINO-4-NITROPHENOXYETHANOL | 0,1 |
| DISPERSE VIOLET 1 | 0,12 |
| BASIC BROWN 16 | 0,03 |
| 2-NITRO-5-GLYCERYL METHYLANILINE | 0,25 |
| HC VIOLET NO. 1 | 0,042 |
| HC BLUE NO. 2 | 0,7 |
| HC YELLOW NO. 7 | 0,02 |
| HC RED NO. 3 | 0,027 |
| HC YELLOW NO. 10 | 0,06 |
| HC BLUE NO. 14 | 0,5 |
| CHLORHEXIDINEDIHYDROCHLORIDE | 0,05 |
| METHYLPARABEN | 0,3 |
| SCLEROTIUM GUM | 1 |
| AMODIMETHICONE (and) TRIDECETH-6 (and) CETRIMONIUM CHLORIDE | 1,8 |
| PROPYLENE GLYCOL | 2 |
| CETEARYL ALCOHOL | 7 |
| BEHENTRIMONIUM CHLORIDE | 4 |
| COCAMIDOPROPYL BETAINE | 10 |
| ETHANOLAMINE | QS pH 9.6 |
| CITRIC ACID | |
| AQUA | QSP |

Cette composition est ensuite appliquée sur des mèches de cheveux gris naturels à 90% de blancs à température ambiante. Après 30 minutes, les mèches de cheveux sont rincées à l'eau courante puis séchées.

Les mèches ont été teintes dans une nuance Châtain Clair peu sélective et de bonne intensité. Les cheveux sont doux.

### EXEMPLE 3

| | Exemple 3 |
|---|---|
| 4-AMINO-3-NITROPHENOL | 0,085 |
| 3-METHYLAMINO-4-NITROPHENOXYETHANOL | 0,021 |
| BASIC YELLOW 57 | 0,1 |
| HC RED NO. 3 | 0,017 |
| HC YELLOW NO. 9 | 0,035 |
| HC ORANGE NO. 2 | 0,061 |
| CHLORHEXIDINE DIHYDROCHLORIDE | 0,05 |
| METHYLPARABEN | 0,3 |
| SCLEROTIUM GUM | 1 |
| AMODIMETHICONE (and) TRIDECETH-6 (and) CETRIMONIUM CHLORIDE | 1,8 |
| PROPYLENE GLYCOL | 2 |
| CETEARYL ALCOHOL | 7 |
| BEHENTRIMONIUM CHLORIDE | 4 |
| COCAMI DOPROPYL BETAINE | 10,53 |
| ETHANOLAMINE | QS pH 6.7 |
| CITRIC ACID | |
| AQUA | QSP |

Cette composition est ensuite appliquée sur des mèches de cheveux gris naturels à 90% de blancs à température ambiante. Après 30 minutes, les mèches de cheveux sont rincées à l'eau courante puis séchées.

Les mèches ont été teintes dans une nuance Doré intense et peu sélective. Les cheveux sont doux.

Pour chacun de ces exemples, le même type de résultat est obtenu lorsqu'on remplace les 1% de Sclerotium gum par 1% de gomme de xanthane.

### Exemples 4 à 7

| | **Exemple 4** | **Comp. Exemple 5** | **Exemple 6** | **Comp. Exemple 7** |
|---|---|---|---|---|
| CHLORHYDRATE DE CHLORHEXIDINE | 0.05 | 0.05 | 0.05 | 0.05 |
| P-HYDROXYBENZOATE DE METHYLE | 0.3 | 0.3 | 0.3 | 0.3 |
| PARFUM | 0.5 | 0.5 | 0.5 | 0.5 |
| POLYGLUCOSE LINEAIRE (SCLEROTIUM GUM) | 1 | 1 | 1 | 1 |
| DOW CORNING 2-8299 CATIONIC EMULSION | 1.8 | 1.8 | 1.8 | 1.8 |
| PROPYLENE GLYCOL | 2 | 2 | 2 | 2 |
| ALCOOL CETYLSTEARYLIQUE (C16/C18 50/50) | 7 | 7 | 7 | 7 |
| CHLORURE DE BEHENYL TRIMETHYL AMMONIUM (GENAMIN KDMP A 79% MA) | 4 | 4 | 4 | 4 |
| COCOYL AMIDOPROPYL BETAINE EN SOLUTION AQUEUSE (TEGO BETAIN F 50 A 38% MA) | 10 | ---- | 10 | --- |
| ALCOOL LAURIQUE OXYETHYLENE (12 OE) | --- | 3.8 | --- | 3.8 |
| 1-HYDROXY-3-NITRO-4-BETA-HYDROXYETHYLAMINO-BENZENE | 0.1 | 0.1 | --- | --- |
| 1-HYDROXY-3-NITRO-4-AMINO-BENZENE | | | 0,085 | 0,085 |
| 1-BETA-HYDROXYETHYLOXY-3-METHYLAMINO-4-NITRO-BENZENE | | | 0,021 | 0,021 |
| 1-(BETA-HYDROXYETHYLAMINO)-2-NITRO-4-AMINOBENZENE | | | 0,017 | 0,017 |
| CHLORHYDRATE DE 1-METHOXY-3(BETA-AMINOETHYL)AMINO-4-NITRO-BENZENE | | | 0,035 | 0,035 |
| 2-[4-[(2-AMINOETHYL)AMINO]-3-NITROPHENOXY]ETHANOL | | | 0,061 | 0,061 |
| BASIC YELLOW 57 | | | 0,1 | 0,1 |
| EAU DESIONISEE | QS | QS | QS | QS |
| MONOETHANOLAMINE PURE | QS pH 6.7 | | | |
| ACIDE CITRIQUE | | | | |

Ces compositions sont appliquées sur des mèches de cheveux gris naturels à 90% de blancs à température ambiante. Après 30 minutes, les mèches de cheveux sont rincées à l'eau courante puis séchées.

### DETERMINATION DE LA COULEUR

La coloration des cheveux est évaluée dans le système L*a*b* system, avec un spectrophotometre Konica Minolta CM2006d / A 1005556.

Dans ce système, L représente l'intensité, plus la valeur de L* est faible, plus la coloration obtenue est intense. La chromaticité est mesurée par les valeurs a* et b*, a* représentant l'axe rouge/vert et b* l'axe jaune/bleu

Les résultats des mesures colorimétriques sont les suivantes :

| **Résultats colorimétriques** | **L*** | **a*** | **b*** |
|---|---|---|---|
| Exemple 1 | **42,17** | 24,93 | 16,9 |
| Exemple 2 | **45,47** | 30,83 | 21,02 |
| Exemple 3 | **40,37** | 18,73 | 33,09 |
| Exemple 4 | **43,2** | 22,78 | 37,82 |

Ces résultats montrent qu'avec les compositions de la présente invention, on obtient une coloration des cheveux plus intenses qu'avec les compositions comparatives dans lesquels le tensio-actif amphotère a été remplacé par un tensio-actif non ionique.

## Revendications

1. Composition de coloration des fibres kératiniques comprenant au moins un colorant direct, au moins un biohétéropolysaccharide, au moins un tensio-actif cationique et au moins un tensio-actif amphotère choisi parmi les alkyl (C₈-C₂₀) bétaïnes, les alkyl (C₈-C₂₀) amidoalkyl (C₁-C₆) bétaïnes ou les alkyl (C₈-C₂₀) amidoalkyl (C₁-C₆) sulfobétaïnes.

2. Composition selon la revendication 1 dans laquelle le colorant direct est choisi parmi les colorants directs nitrés benzéniques neutres, acides ou cationiques, les colorants directs azoïques neutres acides ou cationiques, les colorants directs quinoniques, les colorants directs aziniques, les colorants directs triarylméthaniques, les colorants directs indoaminiques et les colorants directs naturels.

3. Composition selon la revendication 1 ou 2 dans laquelle la quantité de colorants directs est comprise entre 0,001 à 20% en poids du poids total de la composition, préférentiellement entre 0,005 à 10% en poids.

4. Composition selon l'une quelconque des revendications précédentes dans laquelle le biohétéropolysaccharide est choisi parmi les gommes de xanthane, les gommes de scléroglucane, les gommes de gellane, les gommes de pullulane, les gommes de Curdlar, les gommes de grifolane, les gommes de lentinane, les gommes de Schizophyllane; les gommes de spirulinane et les gommes de krestine.

5. Composition selon l'une quelconque des revendications précédentes dans laquelle le biohétéropolysaccharide est une gomme de xanthane.

6. Composition selon l'une quelconque des revendications précédentes dans laquelle le biohétéropolysaccharide est une gomme de schléroglucane (sclerotium gum).

7. Composition selon l'une quelconque des revendications précédentes dans laquelle la quantité en biohétéropolysaccharide est comprise entre 0.01 et 10 % en poids du poids total de la composition, de préférence entre 0.1 et 4 %.

8. Composition selon l'une quelconque des revendications précédentes dans laquelle le ou les tensio-actifs cationiques sont choisis parmi les sels d'amines grasses primaires, secondaires ou tertiaires, les sels d'ammonium quaternaire, et leurs mélanges.

9. Composition selon la revendication 7 dans laquelle le tensio-actif cationique est choisi parmi le chlorure de béhényltriméthylammonium, le chlorure de cétyltriméthylammonium, le quaternium-83, le quatemium-87, le chlorure de béhénylamidopropyl 2,3-dihydroxypropyl diméthyl ammonium, le chlorure de palmitylamidopropyltriméthylammonium et la stéaramidopropyldiméthylamine.

10. Composition selon l'une quelconque des revendications précédentes dans laquelle la quantité en tensio-actif cationique est comprise entre 0,01 à 10 % en poids, de préférence de 0,1 à 4 % en poids par rapport au poids total de la composition.

11. Composition selon l'une quelconque des revendications précédentes dans laquelle le ou les tensioactifs amphotères sont présents en quantité variant de 0,01 à 20 % en poids, de préférence de 5 à 15 % en poids par rapport au poids total de la composition.

12. Composition selon l'une quelconque des revendications précédentes dans laquelle le rapport pondéral tensioactif cationique/biohétéropolysaccharides est supérieur à 1.

13. Composition selon l'une quelconque des revendication précédentes comprenant au moins une base d'oxydation et/ou au moins un coupleur.

14. Composition selon l'une quelconque des revendications précédentes comprenant au moins un agent oxydant.

15. Procédé de coloration des fibres kératiniques dans lequel on applique sur les fibres la composition telle que définie à l'une quelconque des revendications 1 à 13, pendant un temps suffisant pour développer la coloration désirée.

## Patentansprüche

1. Zusammensetzung zum Färben von Keratinfasern, die mindestens einen Direktfarbstoff, mindestens ein Bioheteropolysaccharid, mindestens einen kationischen grenzflächenaktiven Stoff und mindestens einen amphoteren grenzflächenaktiven Stoff enthält, wobei das amphoteren grenzflächenaktiven Stoff unter den alkyl (C₈-C₂₀) betainen, den alkyl (C₈-C₂₀) amidoalkyl (C₁-C₆) betainen oder den alkyl (C₈-C₂₀) amidoalkyl (C₁-C₆) sulfobetainen ausgewählt ist.

2. Zusammensetzung nach Anspruch 1, wobei der Direktfarbstoff unter den neutralen, sauren oder kationischen, nitrierten Direktfarbstoffen auf Benzolbasis, neutralen, sauren oder kationischen, direktziehenden Azofarbstoffen, direktziehenden Chinonfarbstoffen, direktziehenden Azinfarbstoffen, direktziehenden Triarylmethanfarbstoffen, direktziehenden Indoaminfarbstoffen und direktziehenden natürlichen Farbstoffen ausgewählt ist.

3. Zusammensetzung nach Anspruch 1 oder 2, wobei der Mengenanteil der Direktfarbstoffe im Bereich von 0,001 bis 20 Gew.-% des Gesamtgewichts der Zusammensetzung und vorzugsweise im Bereich von 0,005 bis 10 Gew.-% liegt.

4. Zusammensetzung nach einem der vorhergehenden Ansprüche, wobei das Bioheteropolysaccharid unter den Xanthanen, Scleroglucanen, Gellanen, Pullulanen, Curdlanen, Grifolanen, Lentinanen, Schizophyllanen, Spirulinanen und Krestinen ausgewählt ist.

5. Zusammensetzung nach einem der vorhergehenden Ansprüche, wobei das Bioheteropolysaccharid ein Xanthangummi ist.

6. Zusammensetzung nach einem der vorhergehenden Ansprüche, wobei des Bioheteropolysaccharid ein Scleroglucan (Sclerotium gum) ist.

7. Zusammensetzung nach einem der vorhergehenden Ansprüche, wobei der Mengenanteil des Bioheteropolysaccharids im Bereich von 0,01 bis 10 Gew.-% des Gesamtgewichts der Zusammensetzung und vorzugsweise im Bereich von 0,1 bis 4 Gew.-% liegt.

8. Zusammensetzung nach einem der vorhergehenden Ansprüche, wobei der oder die kationischen grenzflächenaktiven Stoffe unter den primären, sekundären oder tertiären Salzen von Fettaminen, quartären Ammoniumsalzen und deren Gemischen ausgewählt sind.

9. Zusammensetzung nach Anspruch 7, wobei der kationische grenzflächenaktive Stoff unter Behenyltrimethylammoniumchlorid, Cetyltrimethylammoniumchlorid, Quaternium-83, Quaternium-87, Behenylamidopropyl-2,3-dihydroxypropyl-dimethyl-ammoniumchlorid, Palmitylamidopropyltrimethylammoniumchlorid und Stearamidopropyldimethylamin ausgewählt ist.

10. Zusammensetzung nach einem der vorhergehenden Ansprüche, wobei der Mengenanteil des kationischen grenzflächenaktiven Stoffes im Bereich von 0,01 bis 10 Gew.-% und vorzugsweise 0.1 bis 4 Gew.-%, bezogen auf daß Gesamtgewicht der Zusammensetzung, liegt.

11. Zusammensetzung nach einem der vorhergehenden Ansprüche, wobei der oder die amphoteren grenzflächenaktiven Stoffe in einem Mengenanteil von 0,01 bis 20 Gew.-% und vorzugsweise 5 bis 15 Gew.-% bezogen auf das Gesamtgewicht der Zusammensetzung, enthalten sind.

12. Zusammensetzung nach einem der vorhergehenden Ansprüche, wobei das Gewichtsverhältnis kationischer grenzflächenaktiver Stoff/Bioheteropolysaccharid über 1 liegt.

13. Zusammensetzung nach einem der vorhergehenden Ansprüche, die mindestens eine Oxidationsbase und/oder mindestens einen Kuppler enthält.

14. Zusammensetzung nach einem der vorhergehenden Ansprüche, die mindestens ein Oxidationsmittel enthält.

15. Verfahren zum Färben von Keratinfasern, bei dem die Zusammensetzung nach einem der Ansprüche 1 bis 14 während einer Zeitspanne, die für die Bildung der gewünschten Farbe ausreichend ist, auf die Fasern aufgebracht wird.

## Claims

1. Composition for dyeing keratin fibres, comprising at least one direct dye, at least one bioheteropolysaccharide, at least one cationic surfactant and at least one amphoteric surfactant chosen from alkyl (C₈-C₂₀) betaines, alkyl (C₈-C₂₀) amidoalkyl (C₁-C₆) betaines or alkyl (C₈-C₂₀) amidoalkyl (C₁-C₆) sulfobetaines.

2. Composition according to Claim 1, in which the direct dye is chosen from neutral, acidic or cationic nitrobenzene direct dyes, neutral, acidic or cationic azo direct dyes, quinone direct dyes, azine direct dyes, triarylmethane direct dyes, indoamine direct dyes and natural direct dyes.

3. Composition according to Claim 1 or 2, in which the amount of direct dyes is between 0.001% and 20% by weight of the total weight of the composition, preferably between 0.005% and 10% by weight.

4. Composition according to any one of the preceding claims, in which the bioheteropolysaccharide is chosen from xanthan gums, sclerotium gums, gellan gums, pullulan gums, curdlan gums, grifolan gums, lentinan gums, schizophyllan gums, spirulinan gums and krestin gums.

5. Composition according to any one of the preceding claims, in which the bioheteropolysaccharide is xanthan gum.

6. Composition according to any one of the preceding claims, in which the bioheteropolysaccharide is a sclerotium gum.

7. Composition according to any one of the preceding claims, in which the amount of bioheteropolysaccharide is between 0.01% and 10% by weight of the total weight of the composition, preferably between 0.1% and 4%.

8. Composition according to any one of the preceding claims, in which the cationic surfactant(s) is (are) chosen from primary, secondary or tertiary fatty amine salts, quaternary ammonium salts, and mixtures thereof.

9. Composition according to Claim 7, in which the cationic surfactant is chosen from behenyl-trimethylammonium chloride, cetyltrimethylammonium chloride, quaternium-83, quaternium-87, behenyl-amidopropyl-2,3-dihydroxypropyldimethylammonium chloride, palmitylamidopropyltrimethylammonium chloride and stearamidopropyldimethylamine.

10. Composition according to any one of the preceding claims, in which the amount of cationic surfactant is between 0.01% and 10% by weight, preferably from 0.1% to 4% by weight, relative to the total weight of the composition.

11. Composition according to any one of the preceding claims, in which the amphoteric surfactant(s) is (are) present in an amount ranging from 0.01% to 20% by weight, preferably from 5% to 15% by weight, relative to the total weight of the composition.

12. Composition according to any one of the preceding claims, in which the cationic surfactant/bio-heteropolysaccharides weight ratio is greater than 1.

13. Composition according to any one of the preceding claims, comprising at least one oxidation base and/or at least one coupler.

14. Composition according to any one of the preceding claims, comprising at least one oxidizing agent.

15. Process for dyeing keratin fibres in which the composition as defined in any one of Claims 1 to 14 is applied to the fibres for a period of time sufficient to develop the desired coloration.
